# EUROPEAN PATENT APPLICATION

(11) **EP 2 014 649 A1**
(43) Date of publication of application: **14.01.2009**
(21) Application number: 07111210.6
(22) Date of filing: 27.06.2007
(51) Int. Cl.: C07D 209/88, A61K 31/403

(54) **Novel amorphous carvedilol dihydrogen phosphate**

(71) Applicant: INKE, S.A., 08755 Castellbisbal (Barcelona) (ES)
(72) Inventor: Barnas Cañero, Rafael, 08924, Santa Coloma de Gramenet (Barcelo (ES); Portell Bueso, Ana, 08201, Sabadell (Barcelona) (ES); Prohens Lopez, Rafel, 08450, Llinars del Vallès (Barcelona) (ES); Puigjaner Vallet, Mª Cristina, 08029, Barcelona (ES); Dalmases Barjoan, Pere, 08980, Sant Feliu de Llobregat (Barcelona) (ES)
(74) Representative: Barlocci, Anna

(57) **Abstract**

The present invention refers to an amorphous of carvedilol dihydrogen phosphate, the processes to obtain it, compositions containing this compound and its use to treat hypertension, congestive heart failure and angina.

## Description

### FIELD OF THE INVENTION

The present invention is directed to a novel amorphous carvedilol dihydrogen phosphate, to the process for its preparation, to compositions containing it and to its use in medicine.

### BACKGROUND ART

Carvedilol or (+)-(Carbazol-4-yloxy)-3-[[2-(o-methoxyphenoxy)ethyl]amino]-2-propanol, is a nonselective β-adrenergic blocker with α₁-blocking activity. Carvedilol is a racemic mixture which has the following formula

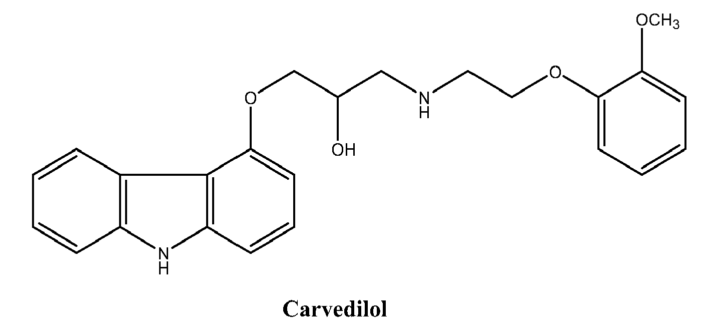

Carvedilol is the object of European patent EP 4.920 B1 and both the racemate and stereoisomers may be obtained according to the processes described therein.

Carvedilol is used for the treatment of hypertension, congestive heart failure and angina.

European patent EP 1.000.027 B1 discloses carvedilol polymorphic forms designated Form I and Form II.

European patent EP 1.406.614 B1 discloses carvedilol polymorphic form III.

International patent application WO 2004/002419 discloses novel crystalline forms of carvedilol dihydrogen phosphate hemihydrate, carvedilol dihydrogen phosphate dihydrate, carvedilol dihydrogen phosphate methanol solvate, carvedilol dihydrogen phosphate and carvedilol hydrogen phosphate.

Difficulties may ensue if the pharmaceutical material contains mixtures of polymorphs, especially if the different polymorphs have varying physical properties. There is substantial interest in amorphous forms of pharmaceuticals because of their different solubility and dissolution rate in comparison to their crystalline counterparts.

In fact, it has been disclosed that the amorphous forms in a number of drugs can exhibit different dissolution characteristics and in some cases, different bioavailability patterns compared to the crystalline form (Konne, T., Chem. Pharm. Bull., 38, 2003 (1990). For some therapeutic indications one bioavailability pattern may be favoured over another. Cefuroxime axetil is a classical example of an amorphous form exhibiting higher bioavailability than a crystalline form.

Thus, it is of interest to have new solid forms of carvedilol.

### SUMMARY OF THE INVENTION

Inventors have surprisingly found that carvedilol dihydrogen phosphate has an amorphous form that shows good stability and appropriate physico-mechanical properties for its manipulation on industrial scale. Consequently, the amorphous carvedilol dihydrogen phosphate of the present invention will be suitable to use as a pharmaceutical and may have advantages over the known crystalline forms, since it has an enhanced solubility.

Thus, a first aspect of the present invention is to provide a novel amorphous form of carvedilol dihydrogen phosphate.

Inventors have also found different processes for the preparation of the amorphous form of carvedilol dihydrogen phosphate, which are advantageous since they allow obtaining the amorphous form of carvedilol dihydrogen phosphate whith a high yield, generally higher than 95%. Thus, a second aspect of the invention provides processes to prepare such amorphous form of carvedilol dihydrogen phosphate.

A third aspect of the invention is directed to pharmaceutical compositions containing amorphous form of carvedilol dihydrogen phosphate.

A fourth aspect of the invention is directed to the use of this compound in the treatment of hypertension, congestive heart failure and angina.

### DESCRIPTION OF THE FIGURES

Figure 1 shows the X-ray powder diffraction pattern (XRPD) of amorphous carvedilol dihydrogen phosphate.
Figure 2 shows the infrared (IR) spectrum of amorphous carvedilol dihydrogen phosphate.
Figure 3 shows the Differential Scanning Calorimetry (DSC) thermogram of amorphous carvedilol dihydrogen phosphate.

### DESCRIPTION OF THE INVENTION

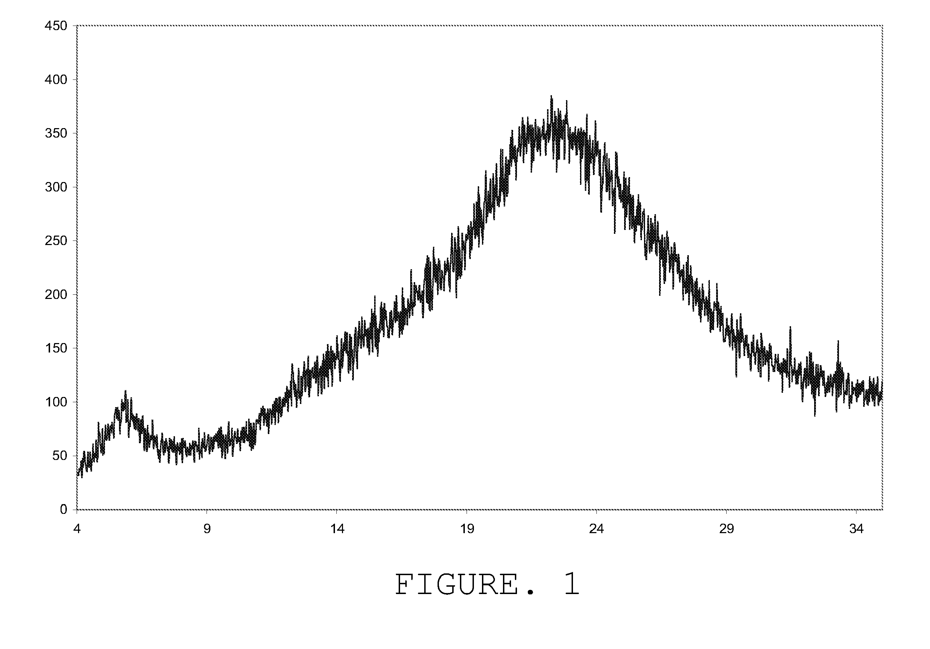

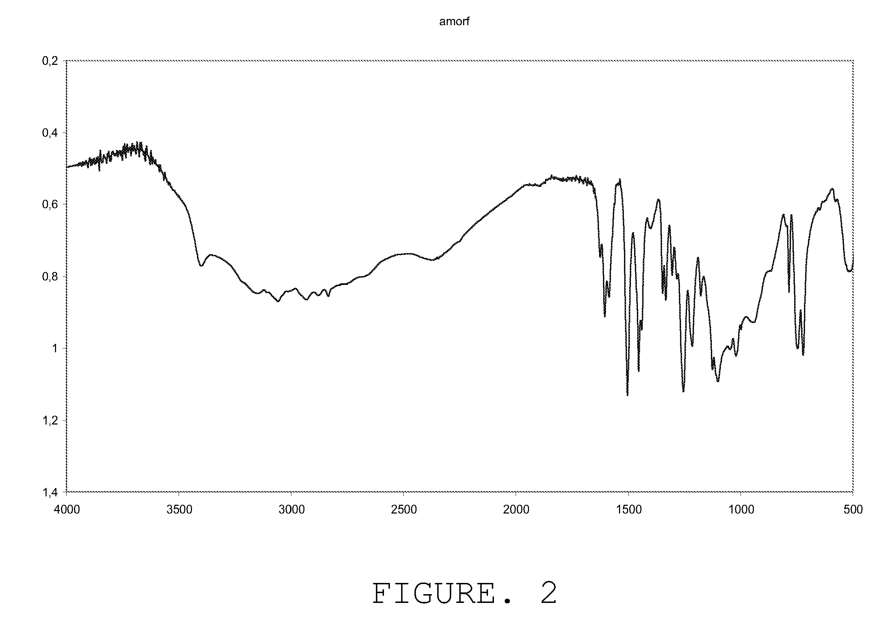

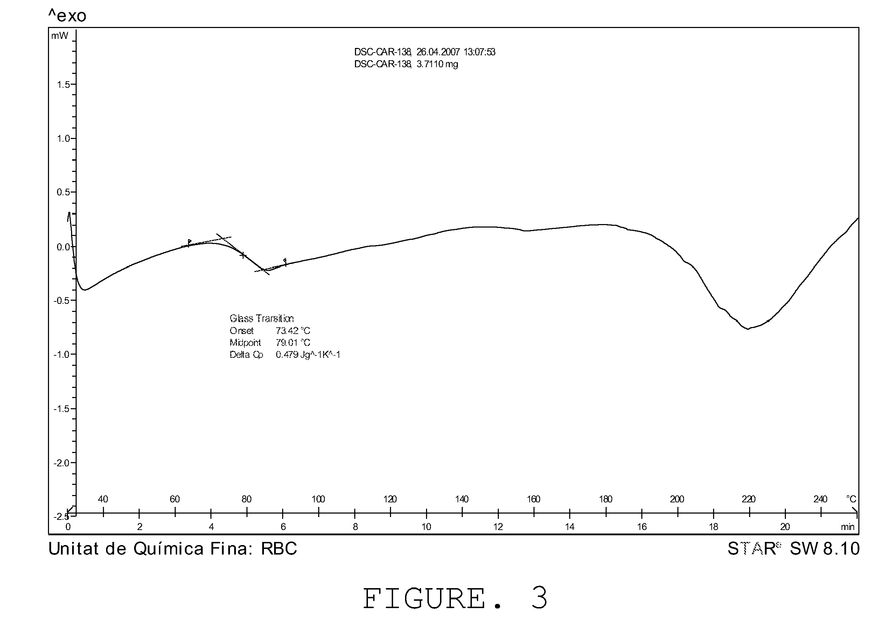

According to the first aspect, the invention provides an amorphous carvedilol dihydrogen phosphate characterized by the X-ray powder diffraction pattern of Figure 1 , the infrared (IR) spectrum (Figure 2), and DSC thermogram wherein no melting point is detected and a glass transition is observed (Figure 3).

XRD patterns were obtained on a analytical X'Pert PRO MPD alpha 1 powder diffractometer, equipped with a CuKα source (λ = 1.54056 Å) and a X'Celerator Detector, operating at 45 kV and 40 mA. Each sample was scanned between 4 and 35° in 2θ, with a step size of 0.017° and a scan rate of 40 s/step.

Differential scanning calorimetry was carried out by means of a Mettler-Toledo DSC-822e calorimeter. Experimental conditions: aluminum crucibles of 40 µl volume, atmosphere of dry nitrogen with 50 ml/min flow rate, heating rate of 10°C/min. The calorimeter was calibrated with indium of 99.99% purity.

Thermogravimetric analyses (TGA) were performed on a Mettler-Toledo TGA-851e thermobalance. Experimental conditions: alumina crucibles of 70 µl volume, atmosphere of dry nitrogen with 50 ml/min flow rate, heating rate of 10°C/min.

FT-IR spectra were recorded on a Bomem MB-120 IR spectrophotometer in KBr pellets, at 1 cm-1 resolution, from 350 to 5000 cm-1.

The glass transition temperature (Tg) is one of the most important features of the amorphous state. The Tg is determined from the jump in heat capacity in the DSC curve. The glass transition corresponds to a change in physical state from a glassy material to a more fluid rubbery state. Knowledge of the Tg is of major importance for storage stability. The inventors of the present application have found that after drying the amorphous carvedilol dihydrogen phosphate of the present invention at 90°C, a glass transition is observed in the DSC at 79°C. Therefore, the amorphous carvedilol dihydrogen phosphate is stable at room temperature.

Moreover, the inventors of the present patent application have found that the amorphous carvedilol dihydrogen phosphate of the invention is stable when tested at room temperature (20-25°C) and relative humidity of 43% in open dish for 10 days.

In another aspect, the present invention provides processes for preparing the above amorphous carvedilol dihydrogen phosphate.

In a first embodiment, the above amorphous carvedilol dihydrogen phosphate is obtained through a process that comprises:
a) providing a suspension of carvedilol in an organic solvent selected from the group consisting of aliphatic (C₂-C₈) ethers, aliphatic (C₁-C₆) alcohols, aliphatic (C₁-C₃) chlorides, and acetonitrile;
b) heating to get a solution;
c) adding phosphoric acid and cooling; and
d) recovery of the precipitated amorphous carvedilol dihydrogen phosphate.

Preferably, said organic solvent is selected from tetrahydrofuran, isopropanol, dichloromethane and acetonitrile. More preferably, acetonitrile, and isopropanol.

In a particular embodiment, the cooling step is performed without stirring.

In a second embodiment, amorphous carvedilol dihydrogen phosphate can also be prepared from anhydrous, hemihydrate and dihydrate carvedilol dihydrogen phosphate by means of a process that comprises:
a) providing a suspension of anhydrous, hemihydrate or dihydrate carvedilol dihydrogen phosphate in an organic solvent selected from aliphatic (C₁-C₆)alcohols and an aprotic polar solvent;
b) heating to get a solution;
c) cooling; and
d) recovery of the precipitated amorphous carvedilol dihydrogen phosphate.

In a particular embodiment, the cooling step is performed without stirring.

In a third embodiment, amorphous carvedilol dihydrogen phosphate is obtained through a process that comprises:
a) providing a solution of carvedilol in an organic solvent selected from aliphatic (C₁-C₆)alcohols and an aprotic polar solvent;
b) adding phosphoric acid;
c) precipitating the amorphous carvedilol dihydrogen phosphate by addition of an antisolvent selected from the group consisting of aliphatic (C₂-C₆) alcohols, aliphatic (C₂-C₈) ethers, aliphatic (C₁-C₃) chlorides, (C₆-C₈) aromatic hydrocarbons; (C₅-C₆) cycloalkanes, (C₃-C₆) -ketones (C1-C4) -alkyl acetate, acetonitrile, and water; and
d) recovery of the amorphous carvedilol dihydrogen phosphate.

In a particular embodiment, after the addition of the antisolvent, the stirring is stopped.

In a fourth embodiment, amorphous carvedilol dihydrogen phosphate can also be prepared from anhydrous, hemihydrate and dihydrate carvedilol dihydrogen phosphate by means of a process that comprises:
a) providing a solution of anhydrous, hemihydrate or dihydrate carvedilol dihydrogen phosphate in an organic solvent selected from aliphatic (C₁-C₆)alcohols and an aprotic polar solvent;
b) precipitating the amorphous carvedilol dihydrogen phosphate by addition of an antisolvent selected from the group consisting of aliphatic (C₂-C₆)alcohols, aliphatic (C₂-C₈)ethers, aliphatic (C₁-C₃) chlorides, (C₆-C₈) aromatic hydrocarbons; (C₅-C₆) cycloalkanes, (C₃-C₆) -ketones (C₁-C₄) -alkyl acetate, acetonitrile, and water; and
c) recovery of the amorphous carvedilol dihydrogen phosphate.

In a particular embodiment, after the addition of the antisolvent, the stirring is stopped.

In embodiments second, third and fourth preferably, the organic solvent is methanol or a polar aprotic solvent selected from the group of dimethyl sulfoxide (DMSO), N,N-dimethylformamide (DMF), N-methylpirrolidone and N,N-dimethylacetamide, preferably, dimethyl sulfoxide and N,N-dimethylformamide.

In embodiments third and fourth, preferably the antisolvent is selected from the group consisting of ethanol, isopropanol, acetonitrile, dioxane, tetrahydrofuran, ethyl ether (Et₂O), acetone, water, ethyl acetate (AcOEt), toluene, dichloromethane, pentane, xylene and chloroform.

Generally, a quick/sudden precipitation of the product favours the formation of the amorphous form. The amorphous carvedilol dihydrogen phosphate can precipitate in form of an oil which subsequently solidifies. There are several ways to favour the formation of the amorphous form, for instance, carrying out the cooling step without agitation, precipitating by addition of an antisolvent, or varying the rate of cooling. Alternatively, the amorphous form of the invention may be obtained by freeze drying or spray drying. Moreover, the amorphous carvedilol of the invention can also be stabilized in pharmaceutical compositions by the use during the preparation process of said amorphous carvedilol of crystallisation inhibitors such as polyvinylpyrrolidone or polyethylene glycols in solid dispersions or by formation of complexes with cyclodextrins.

The most appropriate conditions for carrying out said processes vary depending on other parameters considered by the expert skilled in the art, such as, for instance, the concentrations of starting material, rate of cooling, and the like, which can vary depending on the solvent used. These conditions can be easily determined by the skilled person with the help of the teachings of the examples given below.

Also subject-matter of the present invention is a pharmaceutical composition comprising a therapeutically effective amount of the amorphous form of carvedilol phosphate defined above, together with appropriate amount of pharmaceutically acceptable excipients or carriers.

The compositions provided by the present invention can be administered by any suitable route.

As it is said above it is part of the present invention the use of amorphous carvedilol dihydrogen phosphate for the manufacture of a drug for the treatment hypertension, congestive heart failure or angina. The invention also relates to a method of treatment of a mammal, including a human, suffering from hypertension, congestive heart failure or angina. Said method comprises the administration to said patient of a therapeutically effective amount of amorphous carvedilol dihydrogen phosphate defined above, together with pharmaceutically acceptable excipients or carriers.

The following examples are displayed to illustrate the aspects of the present invention. They do not intend to limit in any way the scope of the invention defined in the present description.

### EXAMPLES

### Example 1

2.0 g (4.9 mmol) of carvedilol are suspended in 28 mL acetonitrile. The suspension is then stirred and heated at 70°C. 0.322 mL (4.9 mmol) of 85% phosphoric acid are slowly added. Then the stirring is stopped and the mixture is left to cool slowly to 20-22°C. The resulting solid is recovered by decantation and vacuum dried at 50°C. 2.4 g of amorphous carvedilol dihydrogen phosphate (97%) are obtained.

### Example 2

100 mg (0.19 mmol) of carvedilol dihydrogen phosphate hemihydrate are dissolved in 0.4 mL of methanol and stirred at 70°C. The stirring is then stopped and the mixture is left to cool quickly to 20-22°C. The obtained solid is recovered by decantation and it is vacuum dried at 50°C. 95 mg of amorphous carvedilol dihydrogen phosphate (95%) are obtained.

### Example 3

50 mg (0.12 mmol) of carvedilol are dissolved in 0.2 mL of DMF at room temperature. 8 µL (0.12 mmol) of 85% phosphoric acid are slowly added. The resulting solution is stirred and 1.6 mL of isopropanol is added. Then the stirring is stopped and the obtained solid is recovered by decantation and it is vacuum dried at 50°C. 48 mg of amorphous carvedilol dihydrogen phosphate (96%) are obtained.

### Example 4

50 mg (0.10 mmol) of carvedilol dihydrogen phosphate hemihydrate are dissolved in 0.2 mL of DMF at room temperature. The resulting solution is stirred and 1.6 mL of isopropanol is added. Then the stirring is stopped and the obtained solid is recovered by decantation and it is vacuum dried at 50°C. 48 mg of amorphous carvedilol dihydrogen phosphate (96%) are obtained.

## Claims

1. Amorphous form of carvedilol dihydrogen phosphate

2. Amorphous form of carvedilol dihydrogen phosphate according to claim 1, having an X-ray powder diffraction pattern spectrum substantially as shown in Figure 1, an Infrared spectrum substantially as shown in Figure 2, and/or a Differential Scanning Calorimetry trace substantially as shown in Figure 3.

3. Amorphous form of carvedilol dihydrogen phosphate as defined in any of the claims 1-2, for use as a medicament.

4. A process for the preparation of an amorphous form of carvedilol dihydrogen phosphate as defined in any of the claims 1-2, comprising:
a) providing a suspension of carvedilol in an organic solvent selected from the group consisting of aliphatic (C₂-C₈) ethers, aliphatic (C₁-C₆) alcohols, aliphatic (C₁-C₃) chlorides, and acetonitrile;
b) heating to get a solution;
c) adding phosphoric acid and cooling; and
d) recovery of the precipitated amorphous carvedilol dihydrogen phosphate.

5. The process according to claim 4, **characterized in that** the organic solvent is selected from tetrahydrofuran, isopropanol, dichloromethane, and acetonitrile.

6. The process according to claim 5, wherein the solvent is isopropanol or acetonitrile.

7. A process for the preparation of an amorphous form of carvedilol dihydrogen phosphate as defined in any of the claims 1-2, comprising:
a) providing a suspension of anhydrous, hemihydrate or dihydrate carvedilol dihydrogen phosphate in an organic solvent selected from aliphatic (C₁-C₆)alcohols and an aprotic polar solvent;
b) heating to get a solution;
c) cooling; and
d) recovery of the precipitated amorphous carvedilol dihydrogen phosphate.

8. A process for the preparation of an amorphous form of carvedilol dihydrogen phosphate as defined in any of the claims 1-2, comprising:
a) providing a solution of carvedilol in an organic solvent selected from aliphatic (C₁-C₆)alcohols and an aprotic polar solvent;
b) adding phosphoric acid;
c) precipitating the amorphous carvedilol dihydrogen phosphate by addition of an antisolvent selected from the group consisting of aliphatic (C₂-C₆) alcohols, aliphatic (C₂-C₈) ethers, aliphatic (C₁-C₃) chlorides, (C₆-C₈) aromatic hydrocarbons; (C₅-C₆) cycloalkanes, (C₃-C₆) -ketones (C₁-C₄) -alkyl acetate, acetonitrile, and water; and
d) recovery of the amorphous carvedilol dihydrogen phosphate.

9. A process for the preparation of an amorphous form of carvedilol dihydrogen phosphate as defined in any of the claims 1-2, comprising:
a) providing a solution of anhydrous, hemihydrate or dihydrate carvedilol dihydrogen phosphate in an organic solvent selected from aliphatic (C₁-C₆)alcohols and an aprotic polar solvent;
b) precipitating the amorphous carvedilol dihydrogen phosphate by addition of an antisolvent selected from the group consisting of aliphatic (C₂-C₆) alcohols, aliphatic (C₂-C₈) ethers, aliphatic (C₁-C₃) chlorides, (C₆-C₈) aromatic hydrocarbons; (C₅-C₆) cycloalkanes, (C₃-C₆) -ketones (C₁-C₄) -alkyl acetate, acetonitrile, and water; and
c) recovery of the amorphous carvedilol dihydrogen phosphate.

10. The process according to any of the claims 7-9, wherein the organic solvent is methanol or a polar aprotic solvent selected from the group of dimethyl sulfoxide, N,N-dimethylformamide, N-methylpirrolidone and N,N-dimethylacetamide.

11. The process according to any of the claims 8-10, wherein the antisolvent is selected from the group comprising ethanol, isopropanol, acetonitrile, dioxane, tetrahydrofuran, ethyl ether, acetone, water, ethyl acetate, toluene, dichloromethane, pentane, xylene, and chloroform.

12. A pharmaceutical composition comprising a therapeutically effective amount of the amorphous form of carvedilol dihydrogen phosphate as defined in any of the claims 1-2, together with appropriate amount of pharmaceutically acceptable excipients or carriers.

13. Use of amorphous carvedilol dihydrogen phosphate as defined in any of the claims 1-2 for the manufacture of a medicament for the treatment of a disease selected from the group consisting of hypertension, congestive heart failure, and angina.
